# EUROPEAN PATENT APPLICATION

(11) **EP 3 861 991 A1**
(43) Date of publication of application: **11.08.2021**
(21) Application number: 21155272.4
(22) Date of filing: 04.02.2021
(51) Int. Cl.: A61K 31/05, A61K 31/365, A61K 31/198, A61K 31/375, A61K 31/4172, A61K 31/4415, A61K 31/51, A61K 31/592, A61K 31/593, A61K 31/7048, A61P 17/02

(54) **COMPOSITION FOR PREVENTING AND TREATING ACUTE AND CHRONIC WOUNDS**

(30) Priority: 04.02.2020 IT 202000002152
(71) Applicant: Foodar Advanced Research S.r.l., 20812 Limbiate (IT)
(72) Inventor: BERTOGLIO-CRUZAT, Juan Carlos, 5090000 VALDIVIA, ILSA TEJA (CL); DAVINELLI, Sergio, 86100 CAMPOBASSO (IT); IORIO, Eugenio Luigi, 34100 SALERNO (IT); POGGI, Angelo, 20058 VILLA SANTA (IT); SCAPAGNINI, Giovanni, 95131 CATANIA (IT); VICENTINI, Tito Giorgio, 20031 CESANO MADERNO (IT)
(74) Representative: Coppo, Alessandro

(57) **Abstract**

A composition combining andrographolide and hydroxytyrosol is provided for the prevention and/or treatment of acute and/or chronic wounds; an injury or lesion of the skin; ulcers or ulceration; or for accelerating the physiological process of wound healing. The composition induces the collagen synthesis in human skin fibroblast and accelerate the physiological process of wound healing. The composition may be in a form suitable for oral administration or topical application.

## Description

### FIELD OF THE INVENTION

The present invention relates to a composition for preventing and treating acute and chronic wounds.

The present invention originates in the pharmaceutical and nutritional field. Specifically, the present invention relates to a food supplement or nutritional food for specific curative aims.

### BACKGROUND OF THE INVENTION

Wound healing is a complex physiological process aimed at restoring a tissue, typically skin, damaged by an injury or lesion. This process is critical in maintaining the barrier function of skin.

Tissue regeneration and repair processes occur after the onset of a tissue lesion originating from a trauma or resulting from a specific pathological condition breaking the physical continuity of the tissue, typically skin. After a skin lesion occurs, regeneration and repair occur in the same tissue.

The tissue repair consists in the substitution of specialized structures brought about by the deposition of collagen and regeneration involving the process of cell proliferation and posterior differentiation through preexisting cells in the tissue and/or stem cells.

Tissue repair is a simple linear process in which the growth factors cause cell proliferation, thus leading to an integration of dynamic changes that involve soluble mediators, blood cells, the production of the extracellular matrix, and the proliferation of parenchymal cells.

Cell and biochemical events in wound repair comprise the following stages: inflammatory reaction, cell proliferation and synthesis of the elements which make up the extracellular matrix, and the posterior period, called remodeling.

These stages are not mutually excluding, but rather overlap over time.

With numerous disease processes, the cascade of events involved in wound healing can be affected, resulting in chronic, non-healing wounds that subject the patient to significant discomfort and distress while draining the medical system of an enormous amount of resources.

Aside from the burden of a chronic wound, even simple wounds originating from minor events such as outpatient surgeries require proper attention and care, and understandably, patients may be concerned about possible resultant scarring.

At present, many products are available for accelerating or improving the healing of wounds.

A first family of products includes compositions for topical application containing one or more ingredients accelerating the natural process of tissue repair dispersed in a suitable carrier. Certain products for topical use are aimed at preventing or stopping blood loss as a result of the wound, and preventing infection of the wound in an attempt to promote healing.

Other products clears the wound area and include in the formulation enzymatic agents such as collagenase, papain-urea and bromelain as active ingredient Additional products for topical application exerts a reparative action on skin.

In many cases the control of the wound closure is reliant upon the initial dressing and wound management via the addition of antimicrobial agents to the wound site. A number of dressings are used to clear the site of extraneous fluid/tissue either by absorption or using vacuum-assisted closure. A common disadvantage of dressings of this nature is the need to retain the dressing in position, which may not be possible depending on wound site.

The addition of silver as an antimicrobial agent to dressings has been extensively used, with a number of products on the market, but the efficacy of these products is mixed. Skin substitutes offer a method for temporary wound coverage but ultimately autografting is required due to the survival of the cultured sheets of cells.

A number of targets exist for the acceleration of wound healing, these include growth factors, oxidised regenerated cellulose/collagen matrices, adenosine A_{2A} agonists and recombinant lactoferrin. These act in a variety of ways; the collagen matrix acts to inhibit the negative impact of proteases and oxygen free radicals whilst promoting the activity of growth factors within the wound area; the lactoferrin acts to promote IL-18 up-regulation within the wound.

The management of wounds, make use of treatments such as the application of dressings typically gauze dressings, which may be used alone or impregnated with a variety of anti-infective agents, alginates, hydrocolloids, synthetic composite membranes, transparent films or honey, application of artificial skin, application of allografts typically bovine or porcine allografts or application of ointments or creams typically containing silver based compounds as anti-infective agents.

A second family of compounds aimed at accelerating the healing of wounds or preventing the occurrence of chronic wounds include formulation for oral administration containing specific nutrients especially proteins, vitamins, especially vitamins A and C and other micronutrients such as zinc.

Nutraceuticals and plant derived compounds for oral administration are also available to improve wound healing and prevent or treat non healing wounds. These products are also used as secondary therapy in treating the occurrence of wounds associated with an underlying process ranging from diabetes to cancer to malnutrition.

The absence of a single universally accepted method for accelerating the healing of wounds is indicative of the need for novel medicaments and methods by which such acceleration may be effected.

Even in the case of the available therapies, there is the need for alternative or improvement in terms of increased efficacy and compliance.

Accordingly, one aim of the present invention resides in providing a composition for accelerating the healing of wounds or preventing or treating chronic wounds. Another aim resides in providing a composition for oral administration, which reduces or prevent the occurrence of chronic healing.

An additional aim of the invention consists in providing a composition either for oral or topical administration for accelerating the healing of wounds.

### SUMMARY OF THE INVENTION

In accordance with certain aspects of the present invention, the inventors found that by combining andrographolide with hydroxyltyrosol, a synergistic acceleration of the healing of wounds or faster closure of a break of an epithelium especially skin or surficial layer of an organ is achieved.

In view of this finding, the present invention provides for a synergistic composition for accelerating the healing of wounds or preventing or treating wounds, said composition comprising andrographolide and hydroxytyrosol in combination and a physiologically acceptable carrier or excipient.

The inventors also found that a suitable source of andrographolide for the uses according to the invention is represented by a plant of Andrographis paniculata. Therefore, according to certain embodiments of the invention the combination composition is obtained by a vegetal extract of Andrographis paniculata.

Typically, the active ingredients of the combination composition of the invention are provided in a pharmaceutically or nutritionally effective amount.

The composition of the invention may be a pharmaceutical composition, a nutraceutical, a dietary supplement which can be introduced into the dietary regimen of an individual with a wound or which is in need of preventing chronic wounds or accelerating would healing.

In accordance with another aspect, the invention relates to a composition comprising a combination of an andrographolide and hydroxytyrosol and a physiologically acceptable carrier or excipient for use in the prevention and/or treatment of acute and/or chronic wounds.

In accordance with another aspect, a composition is provided comprising andrographolide and hydroxytyrosol and a physiologically acceptable carrier or excipient, for use in wound healing.

In accordance with an additional aspect, the invention related to a method for stimulating collagen synthesis and/or skin regeneration said method comprising the topical application of a combination composition as herein described on acute and/or chronic wounds. This method is specifically aimed at restoring skin damaged by an injury or lesion.

The inventors found that the combined composition increases the formation of fibroblasts which promote the proliferative phase with collagen synthesis.

The production of collagen and ground substance is crucial for recovering the damaged tissue since it forms the basis for the tissue scaffold of the previous wound area. Meanwhile, endothelial cells enter a growth phase while angiogenesis occurs within the granulation tissue, forming a vascular network supplying this active area of healing. Around 2-3 weeks after, the wound transitions to a remodeling, or maturation phase wherein the collagen type is restored to usual type I, rather than type III seen in a new wound, and the wound tissue matures, resulting in full cross-linking and restoration of a physiological structure.

The present invention is described in details herein below by making reference to the enclosed figures.

### BRIEF DESCRIPTION OF THE DRAWINGS

Some features and advantages of the present invention will become apparent from the accompanying drawings, in which:

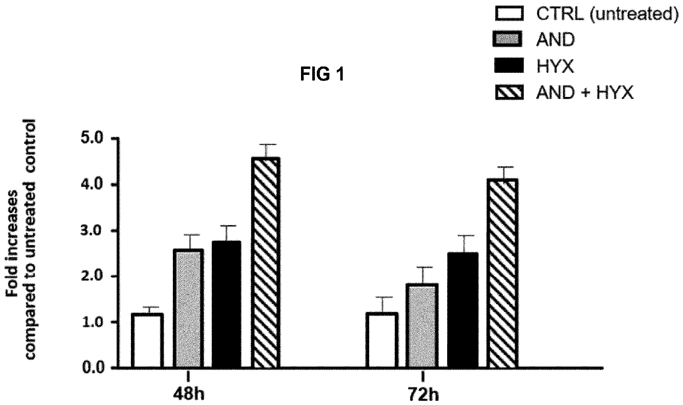

Figure 1 shows bar graphs which illustrates the results of the in vitro comparative test of Example 1 on collagen synthesis in human dermal fibroblast cells (HDF) induced by andrographolide (AND) alone, hydroxytyrosol (HYT) alone, and a combination of AND and HXT. The data shows that AND + HYX treatment induced a 4.7-fold increase at 48hours and 4.2 fold increase at 72 hours in collagen production compared to the untreated control cells.

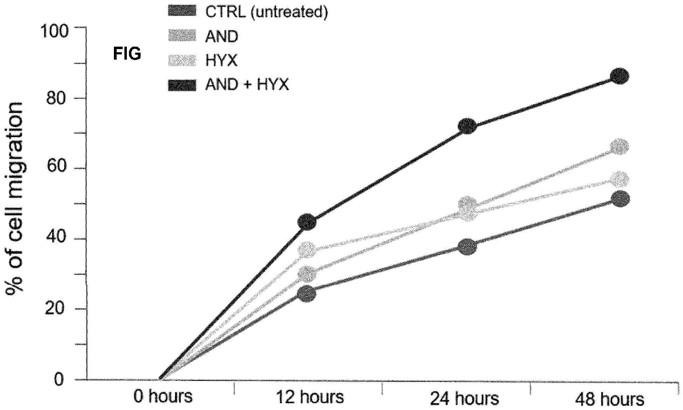

Figure 2 shows graphs reporting the outcome of the test of Example 2 on proliferation and migration of fibroblasts which is indicative of wound closure. The graphs show the percentage of wound closure at different time intervals (12, 24, 48 hours) of AND alone, HYT alone, AND+HYX combination and untreated cells (CTRL). The graphs shows that *AND+HYX combination* induces the faster migration of HDF cells resulting in closure of the gap, created by the scratch, by 93.525% in 48 h. The percentage of cell migration is well lower for the single components (AND; HYX) of the composition.

### DETAILED DESCRITION OF THE INVENTION

In accordance with certain aspects of the present invention, the inventors have discovered that a combination of andrographolide and hydroxytyrosol provides a synergistic stimulating effect on skin fibroblast providing a faster heal of wounds and closure of a break of an epithelium such as skin.

The inventors also found that by combining andrographolide and hydroxytyrosol, an unexpected induction of collagen synthesis in human skin fibroblast is achieved. The combination of active ingredients also increases the reparative process occurring in the healing of wounds and reduce the time of healing of wounds. The authors have evidences these effects by using scratch assay as a primary model.

Accordingly, in a general aspect the invention provides a combination composition for the prevention and/or treatment of acute and/or chronic wounds or for accelerating the physiological process of wound healing in a human individual.

In accordance with a first aspect, the present invention provides a composition for preventing of treating acute and/or chronic wounds and/or for accelerating the physiological process of wound healing as defined in appended claim 1.

Starting from the study of an experimental in vitro biological model which is representative of healing of wounds, the authors found that andrographolide when combined with hydroxytyrosol exert a synergistic reduction of inflammation and cytotoxicity of keratinocytes with benefits in the physiological tissue reparation process in mammals, included animals and human beings.

Embodiments of the composition of the invention are defined in appended claims 2-10. The composition of the invention finds application in the prevention and/or treatment of acute and/or chronic wounds, especially those which develop in a human individual affected by diabetes.

As used herein, the term "combination" means that one or more of the active ingredients are added or mixed with one or other ingredients.

The term "combination" is not intended to mean that the active ingredients are associated with each other with the formation of chemical or other types of bonds.

A biologically active ingredient or component of the combination composition of the invention is represented by andrographolide

Andrographolide is an unsaturated trihydroxy lactone having IUPAC name 3-[2-[Decahydro-6-hydroxy-5-(hydroxymethyl)-5,8a-dimethyl-2-methylene-1-napthalenyl]ethylidene]dihydro-4-hydroxy-2(3*H*)-furanone (CAS Number 5508-58-7) and the following formula:

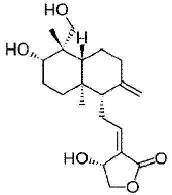

Of the diterpenoids that have been identified and isolated from the plant *A. paniculata,* andrographolide is the most prominent in biological activity, occurrence and quantity. The above referred andrographolide is a naturally occurring biologically active diterpenoid lactones which may be obtained by extraction from a plant such as Andrographis paniculata, a herbaceous plant of the Acanthaceae family. These diterpenoid lactones are distributed in and have been isolated especially from the aerial parts, stem and roots of this plant especially from leaves.

Andrographolide is the main bioactive compound in Andrographis *paniculata.*

The composition of the invention may contain Andrographolide as such or as plant extract. For example, an andrographolide-rich plant extract may be obtained by extraction from a part of Andrographis paniculata such as leaves, root or stem, especially leaves. Preferably, andrographolide is obtained or originates from dried leaves of Andrographis panniculata.

In the alternative, the composition may also contain andrographolide of synthetic origin.

The naturally occurring andrographolide may be obtained through conventional extraction techniques from a portion of a plant containing them, such as *Andrographis paniculata.*

Typically, in the extraction step suitable physiologically acceptable solvents, for example polar organic solvents such as ethanol, or a mixture of ethanol and water may be used.

By way of an example, the Soxhlet extraction is a technique suitable for extracting and separating the relevant andrographolide from a plant source.

For example, a suitable extraction technique comprises the steps of immerging a part of Andrographis paniculata especially leaves in a bath including a physiologically acceptable polar organic solvent, e.g. ethyl alcohol or a mixture water ethyl alcohol, optionally increasing the temperature of the bath to reach a temperature from 30 to 70°C such that the heat applied to the distillation flask reaches the extraction cavity. A suitable alcohol-ethanol may have the concentration as defined hereinbelow.

In certain embodiments, supercritical fluid extraction employing CO₂-ethanol mixture as solvent is used in extracting andrographolide from leaves, preferably dried leaves, of *A. paniculata.* The andrographolide yield increases when the ethanol concentration in the solvent mixtures is increased from 0 to 12.5 mol %.

A suitable extraction is also obtained by the leaf, especially dried leaf such as powder, by cold maceration in a 1:1 mixture of dichloromethane and methanol or ethanol in water with ethanol concentration from 10 to 90%, from 30 to 70% by volume and isolation of andrographolide for example directly from the resulting extract by recrystallization.

A suitable solid-liquid extraction of andrographolide is described by R. Wongkittipong et al. in Solid-liquid extraction of Andrographolide from Plants-Experimental Study, Kinetic Reaction and Model; Sepn. Purifn. Technol. 40:147-154 whose contents is fully incorporated herein by reference.

According to a preferred embodiment, leaves from Andrographis *paniculata* are dried and pulverized to a powder. The extraction is carried out using solution of ethanol in water with ethanol concentration from 10 to 90%, from 30 to 70% by volume. For example, about 10 g of the powder from A. paniculata are added to 100 mL of the extraction solvent then soaked with continuous stirring for 6 h then left for a further 12h without stirring. Suitable solvents used for extraction may be water, and solutions of 30%, 50%, 70% and pure ethanol. The filtrate is collected, concentrated for example with a rotary evaporator, and then dried preferably in a freeze-dryer.

In some embodiments, andrographolide is present in the composition of the invention in an amount from 0.01 to 30% by weight, from 0.1 to 20% by weight, from 0.5 to 10% by weight of the total weight (w/w).

According to some embodiments, the composition of the invention contains andrographolide in an amount from 0.1 to 200 mg, from 1 to 100 mg, from 2 to 50 mg, for example from 3 to 10 mg of dosage unit.

Another biologically active component of the combination composition of the invention is represented by hydroxytyrosol (HYT). This is a phenylethanoid compound provided with antioxidant properties in vitro.

In nature, hydroxytyrosol is found in olive leaf and olive oil, especially in the form of its elenolic acid ester oleuropein and, especially after degradation, in its plain form. Hydroxytyrosol may be drawn from the olive tree and its leaves as a by-product obtained from the manufacturing of olive oil.

According to some embodiments, hydroxytyrosol is of natural origin and may be obtained through conventional production processes.

According to some embodiments, the composition of the invention contains hydroxytyrosol in an amount from 0,1 to 100 mg, from 0.5 to 50 mg, from 1 to 20 mg of unit of dosage.

According to some embodiments the composition of the invention contains hydroxytyrosol in an amount from 0.1 to 95% by weight, from 1 to 90% by weight, from 3 to 60%, from 10 to 30% by weight.

In certain embodiments in the composition the amount of andrographolidesandrographolide to hydroxytyrosol is 2:1.

In certain embodiments the composition comprises an edible and/or physiologically acceptable diluent, excipient or carrier. The physiologically acceptable diluent, excipient or carrier may be chosen based on the intended route of administration of the resulting composition.

The composition may be formulated for oral administration or topical application.

The biologically active substances contained in the composition may be combined or mixed as active ingredients in intimate mixture with an edible carrier and/or an excipient according to the pharmaceutical and food industry or traditional nutritional techniques. As used herein, the term "edible" means edible substances whose use in the formulation of a nutritional or dietary composition for oral administration is approved by the health authorities.

The term "physiologically acceptable" refers to substances that are commonly used in the formulation of nutritional, dietary or pharmaceutical products. A "physiologically acceptable" carrier may be a pharmaceutically acceptable carrier.

The term "carrier" as used herein indicates a medium, excipient, diluent with which the combination of therapeutic or active ingredients is administered.

Any carriers/vehicles and/or excipients suitable for the desired form of preparation for administration to human beings is contemplated for use with the compounds described in the present invention.

In certain embodiments, the composition of the invention comprises additional biologically active substances or active ingredients.

For example, the composition may include one or more vitamins for example vitamin of the B group such as B1, B6, vitamin A, vitamin C, vitamin D.

According to some embodiments, the composition of the invention further comprises one or more micronutrients and/or minerals such as Zn, Mg, K, Na, Fe, Cr, Se, Mn, Ca salts and mixtures thereof.

In certain embodiments the composition also contains amino acids such as valine, lysine, histidine, arginine, leucine and mixtures thereof.

The compositions of the invention are suitable for dietary, nutritional, dietetic or pharmaceutical use in mammals, in particular in human beings.

The composition of the invention can assume a wide variety of forms of preparation, according to the desired route of administration.

The composition of the invention may be in solid, liquid or semiliquid forms.

When the composition of the invention is presented in solid form it may be in the form of a tablet, capsule, powder, granules, toothpaste, oral cream, candy, dissolvable pill or strip, chewing gum, lozenge and powder than can be sprinkled directly in the oral cavity.

The preparations in solid form can comprise one or more excipients/carriers such as for example amides, sugars, microcrystalline cellulose, and optionally diluents, granulation agents, lubricants, ligands, disintegration agents.

Typically the composition is solid form may contain a ligand such as tragacanth, gum, corn starch or gelatin; excipients such as dicalcium phosphate; a disintegration agent such as corn starch, potato starch, alginic acid; a lubricant such as magnesium stearate; a sweetening agent such as sucrose, lactose or saccharin. If desired, the tablets can be coated using traditional techniques.

When the unit form is a capsule, it can contain in addition to the materials of the aforesaid type a liquid carrier such as a fatty oil.

According to certain embodiments the composition of the invention contains an excipient based on cellulose that comprises i) organic esters of cellulose for example selected from cellulose acetate, cellulose propionate, cellulose triacetate, cellulose acetate propionate, cellulose acetate butryate, ii) inorganic esters of cellulose for example selected from nitrocellulose, cellulose sulfate, iii) ethers of cellulose selected from a) cellulose alkyl ethers for example selected from methyl cellulose, ethyl cellulose, ethyl methyl cellulose; b) hydroxyalkyl ethers of cellulose for example selected from hydroxyethyl cellulose, hydroxypropyl cellulose, hydroxyethyl cellulose, hydroxypropyl methyl cellulose, ethyl hydroxyethyl cellulose; c) carboxyalkyl cellulose for example carboxy methyl cellulose, salts thereof and mixtures thereof. In certain embodiments the excipients based on cellulose are crosslinked with physiologically acceptable crosslinking agents.

In some embodiments, the composition of the invention further comprises one or more additional components such as additives, fillers, stabilizers, emulsifiers, texturizers, film-forming agents, plasticizers, wetting agents and thickeners.

Various other materials may be present as coatings or to modify the physical shape of the pharmaceutical unit. For example, the tablets can be coated with shellac, sugar or both. To prevent the breakdown during transit through the upper part of the gastrointestinal tract, the composition may be a formulation with a gastro-resistant or enteric coating.

In the composition there may also be flavouring agents, preservatives, colouring agents and the like.

In certain embodiments the composition of the invention is in solid tablet form.

According to certain embodiments the composition is in a form for the modulated or controlled release of active ingredients. By way of example, the composition may be a delayed release, quick release or slow-fast release formulation. Typically, modulated or controlled release formulations contain one or more excipients based on cellulose, in particular of the type previously described.

In certain embodiments, the composition of the invention comprises as an excipient a hydrogenated fatty acid, preferably having a chain with 3 to 20 carbon atoms, 14 to 18 carbon atoms. A typical example of a hydrogenated fatty acid is hydrogenated palm oil. The composition of the invention may be in liquid form.

When the composition is in liquid form, it may be in the form of a suspension, emulsion, solution, oral spray, mouth wash. In these cases the carrier is liquid and can be selected for example from water, glycols, oils, alcohol and mixtures thereof.

In certain embodiments in which the formulation is in liquid form, e.g. in cases of a syrup or elixir it can contain in addition to the association of active ingredients, saccharose as a sweetening agent, methyl- and propyl-parabens as preservatives, a colouring agent and a flavouring such as cherry or orange flavouring.

The composition of the invention may also be in semiliquid form for example in the form of an emulsion or gel.

The combination compositions may be suitably presented in a single pharmaceutical form and prepared using any of the methods well known in the pharmaceutical or dietary state of the art.

In some embodiments, in the compositions of the present invention, the active ingredients are typically formulated in a dosage unit. The dosage unit can contain from 0.1 to 1,000 mg of each active ingredient per dosage unit for daily administration.

In some embodiments the dose is in the interval from 0.001 % by weight to about 60% by weight of the formulation.

According to some embodiments the composition of the invention is a medicinal product or medicament.

According to some embodiments, the composition of the invention is a nutraceutical, food supplement, a nutritional product or a dietetic product.

In accordance with certain aspects, the invention concerns a method for the prevention or treatment of acute and chronic wounds, wherein the method comprises the simultaneous, separate or sequential administration of a combination of andrographolide and hydroxytyrosol as herein described.

The composition according to the present invention may be topically or systemically administered. Systemic administration is understood to refer to any mode or route of administration that results in effective amounts of extract appearing in the blood or at a site remote from the site of administration.

A preferred systemic administration is the oral route.

Preferably, for the oral administration the composition is in form of a tablet. Preferably, for topical use the composition is in form of a cream or ointment.

The actual amount administered, and the rate and time-course of administration, will depend on the nature and severity of the condition to be treated. Prescription of treatment, e.g. decisions on dosage etc., is ultimately within the responsibility and at the discretion of general practitioners and other medical doctors, and typically takes account of the condition to be treated, the condition of the individual subject, the site of delivery, the method of administration and other factors known to practitioners. The precise dose will depend upon a number of factors, including the form of the composition to be administered.

In certain embodiments the composition is administered or applied daily to a subject.

Unless otherwise defined, all technical and scientific terms used herein have the meaning commonly understood by a person who is skilled in the art in the field of the present invention.

### EXAMPLE 1

Experimental evidence of the activity of the synergistic combination (AND + HYX in pure form) composition of invention.

### Cell culture

Human dermal fibroblasts (HDF) (ATCC, Manassas, VA, USA) were grown in fibroblast basal medium supplemented with fibroblast growth kit-serum free (ATCC).

Fibroblasts are the most abundant cell type in the dermal layer of skin; they are also accessory cells in the inflammatory response. Fibroblasts can produce or respond to a wide variety of cytokines, and these mediators allow fibroblasts and leukocytes to cooperate during complex processes such as wound healing.

Investigating the effect of the combination of the active ingredients Andrographolide and Hydroxytyrosol in HDF cells provides evidence and insight into how the composition impact on wound healing.

AND (andrographolides in pure form, 98% by HPLC) was provided by Sigma-Aldrich code N. (365645)-500mg

HYX (Hydroxytyrosol) in pure form ≥98% by HPLC) was provided by Sigma-Aldrich Code N. H4291-100mg.

### Collagen production

To monitor collagen produced in cell culture supernatants of different conditions, the Sircol Assay (Biocolor, Northern Ireland, UK) was performed.

Supernatants were prepared from the HDF cells unstimulated and treated with 10 µM of AND (andrographolide), HYX (Hydroxytyrosol) or AND + HYX or with pbs as control. Cell-free supernatants were collected after 48 and 72 hours of incubation and assessed for total soluble collagen production according to the manufacturer's protocol. Collagen synthesis was measured in human dermal fibroblast cells. Total collagen production and secretion were significantly up regulated in treated HDF in comparison to untreated control cells. These data showed that AND + HYX treatment induced a 4.7-fold increase at 48 and 4.2 fold increase at 72 hours in collagen production compared to the untreated control cells, as evidenced in Fig. 1.

### EXAMPLE 2

### Fibroblast cell migration and wound closure

Activation, proliferation and migration of fibroblasts are the primary steps in wound healing, where multiple cell types and other micro environmental factors are involved. Scratch assay is a widely applied *In vitro* technique for understanding the wound healing capabilities of medicinally important compounds.

HDF cells were seeded in 6-well plates and were cultured overnight. Cells were then washed with Delbucco's Phosphate Buffered Saline (DPBS) and a scratch was made with a sterile 200µL tip. The detached cells and other cellular debris were removed by washing the cells with DPBS.

The cells were treated with 10 µM of AND (source of Example 1), HYX (source of Example 1), or *AND* + *HYX (1*/*1 ratio, same sources)* and incubated for 24 h. Untreated cells were negative control. The cell migration and morphological changes of cells were observed in the images taken by inverted microscope, equipped with digital camera. The experiments were performed in triplicate (n = 3). The width of the scratch and wound closure at different time intervals (0, 12, 24 and 48hrs) was analyzed by Image J software.

The results indicated that *the AND+HYX combination* closed the gap created by the scratch by 93.525% in 48 h. Percentage wound closure at different time intervals in AND alone, HYX alone or untreated cells are represented in Fig. 2.

AND + HYX combination induced the migration of HDF cells resulting in wound closure.

### EXAMPLE 3

A composition for oral administration comprising

| | |
|---|---|
| Andrographolide | 100mg |
| Hydroxytyrosol | 50 mg |
| Excipients | 3000 mg |

### EXAMPLE 4

A composition for topical application comprising

| | |
|---|---|
| Andrographolide | 25 mg |
| Hydroxytyrosol | 10 mg |
| Excipients suitable for topical application | 3000 mg |

### EXAMPLE 5

Nutritional product (food supplement for special medical needs) in the forms of watersoluble granules for oral administration having the following content for unit of dosage:

| NUTRITIONAL INFORMATION | for 1 sachet (5,5 g) | for 2 sachets (11 g) | for 100 g of product |
|---|---|---|---|
| Energetic value | 7.73 KJ | 15.46 KJ | 140.55 KJ |
| | 1.8 Kcal | 3.6 Kcal | 32.73 Kcal |
| Fats | 0 g | 0 g | 0 g |
| *of which saturated* | 0 g | 0 g | 0 g |
| Carbohydrates | 0,17 g | 0,34 g | 3g |
| *of which sugars* | 0,03 g | 0,06 g | 0,6 g |
| Fibers | 0 g | 0 g | 0 g |
| Proteins* | <0.01 g | <0.01 g | <0.01 g |
| Salt | 0.04 g | 0,08 g | 0,73 g |
| Hydroxytyrosol in pure form 99% by HPLC | 5,5mg | 11 mg | 100mg |
| Andrographolide 98% by HPLC | 11 mg | 22mg | 200mg |
| L-Valine | 0,62 g | 1,25 g | 11,36 g |
| L-Leucine | 1,25 g | 2,5 g | 22,73 g |
| L-Lisine | 0,65 g | 1,3 g | 11,82 g |
| L-Threonine | 350 mg | 700 mg | 6,36 g |
| L-Histidine | 150 mg | 300 mg | 2,73 g |
| L-Cysteine | 100 mg | 200 mg | 1,82 g |
| L-Glutamine | 100 mg | 200 mg | 1,82 g |
| L-Methionine | 75 mg | 150 mg | 1,36 g |
| Glycine | 50 mg | 100 mg | 0,91 g |
| L-Tryptophan | 35 mg | 70 mg | 0,64 g |
| Vitamin C | 35 mg | 70 mg | 0,64 g |
| Zinc | 7,5 mg | 15 mg | 0,14 g |
| HMB | 5 mg | 10 mg | 91 mg |
| Vitamin B1 | 0,25 mg | 0,5 mg | 5 mg |
| Vitamin B6 | 0,2 mg | 0,4 mg | 3,64 mg |
| Vitamin D | 10 mcg (400 Ul) | 20 mcg (800 UI) | 182 mcg (7272 UI) |

### EXAMPLE 6

Topical product in the form of a cream for treating skin ulcerations containing the following ingredients:
Andrographolide, Hydroxytyrosol, Glycerin, Olea Europaea Fruit Oil*, Helianthus Annuus Seed Oil, Zinc Oxide, Cera Alba, Prunus Amygdalus Dulcis Oil, Squalane, Tapioca Starch, Boswellia Serrata extract, Zea Mays Oil, Calendula Officinalis Extract, Lecithin, Tocopherol, Ascorbyl Palmitate, Citric Acid

### EXAMPLE 7

Example of nutritional supplement of "natural source/origin" in the form of a soluble powder to be added to a liquid before oral administration wherein the two active ingredients andrographolides (from leaves) and Hydroxyl tyrosol (from olive oils) are extracts from plants.

| NUTRITIONAL INGREDIENTS | AMOUNT: 100 g |
|---|---|
| Fats | 0,5 g |
| Carbohydrates | 18,9 g |
| Fibers | 2,3 g |
| Proteins (Nx6,25) | 68,5 g |
| Salt | 0,375 g |
| Green tea dry extract | 476,2 mg |
| Vitamin B6 | 8,1 mg |
| L-leucine | 18,1 g |
| L-valine | 6,7 g |
| L-isoleucine | 6,2 g |
| Calcium Hydroxymethylbutyrate | 6,38 g |
| Vitamin D | 10 mcg |
| Dry olive oil extract titred in Hydroxyl tyrosol 20% | 238,1 mg |

## Claims

1. A composition comprising hydroxyltyrosol and andrographolide for use in the prevention and/or treatment of acute and/or chronic wounds, ulcers or ulceration or for accelerating the healing of wounds.

2. The composition for use according to claim 1, wherein the ulcer or ulceration is of an epithelium of a human body.

3. The composition for use according to claim 2, wherein the epithelium is skin.

4. The composition for use according to any one of claims 1-3, wherein the andrographolide is contained in an extract of the plant *Andrographis paniculata.*

5. The composition for use according to claim 4 wherein the extract is obtained by the stem or leaf of *Andrographis paniculata.*

6. The composition for use according to any one of claims 1-5 wherein the wounds are cuts, scratches, bruises, burns or lacerations of skin.

7. The composition for use according to any one of claims 1-6, further comprising a vitamin selected from vitamin B1, B6, vitamin C, vitamin D and mixtures thereof and an aminoacid selected from valine, lysine, histidine, arginine, leucine and mixtures thereof.

8. The composition for use according to any one of the claims 1-7, for oral administration.

9. The composition for use according to claim 8, in solid form of a tablet, capsule, powder, granules, toothpaste, oral cream or gel, candy, dissolvable pill or strip, chewing gum, lozenge or powder or in liquid form of suspension, emulsion, solution, oral spray, mouth wash.

10. A nutritional or food supplement comprising a composition for use according to anyone of claims 1 to 9 and an edible carrier.

11. The composition for use according to any of claims 1-7, for topical application.

12. A composition for use in the treatment of an injury or lesion of the skin by stimulating collagen synthesis and repairing damaged or injured skin, wherein said composition comprises a combination of hydroxyltyrosol and andrographolide and an edible carrier.
